# EUROPÄISCHE PATENTSCHRIFT

(11) **EP 1 512 431 B1**
(45) Veröffentlichungstag und Bekanntmachung des Hinweises auf die Patenterteilung: **27.12.2006**
(21) Anmeldenummer: 04090236.3
(22) Anmeldetag: 16.06.2004
(51) Int. Cl.: A61N 1/37

(54) **Elektrostimulator**
Electrostimulating device
Stimulateur électrique

(30) Priorität: 02.09.2003 DE 10341301
(43) Veröffentlichungstag der Anmeldung: 09.03.2005
(73) Patentinhaber: Biotronik GmbH & Co. KG, 12359 Berlin (DE)
(72) Erfinder: Meier, Jan H., Dr., 91080 Spardorf (DE)
(74) Vertreter: Lindner-Vogt, Karin L.

(56) Entgegenhaltungen:
- EP-A- 0 479 215
- US-A- 4 817 605
- PACE, Bd. 13, Januar 1990 (1990-01), Seiten 69-77, XP008037408

## Beschreibung

Die Erfindung betrifft einen Elektrostimulator mit Elektrodenanschlüssen, die wenigstens zeitweise mit einer Elektrostimulationseinheit und gleichzeitig oder abwechselnd mit einer Detektionseinheit des Elektrostimulators zu verbinden sind. Die Stimulationseinheit ist dabei ausgebildet, Elektrostimulationsimpulse zur Stimulation von Körpergewebe zu generieren und an wenigstens einen der Elektrodenanschlüsse abzugeben. Die Detektionseinheit ist ausgebildet, eine erfolgreiche Stimulation von Körpergewebe anhand eines an wenigstens einem Elektrodenanschluss anliegendem elektrischen Signal zu detektieren. Zu diesem Zweck ist der Elektrostimulator ausgebildet, über den mit der Detektionseinheit verbundenen Elektrodenanschluss ein elektrokardiales Elektrokardiogramm repräsentierendes elektrisches Signal aufzunehmen.

Derartige Elektrostimulatoren sind insbesondere als implantierbare Herzschrittmacher aber auch als Cardioverter/Defibrillator oder als Kombinationsgeräte bekannt.

Solche Elektrostimulatoren dienen vor allem dazu, Elektrostimulationsimpulse an ein Myokard eines Herzens abzugeben, um das Herz dazu zu veranlassen, durch den Elektrostimulationsimpuls angeregt zu kontrahieren. Dazu muss der Elektrostimulationsimpuls über der Reizschwelle des Myocards liegen. Da für einen Elektrostimulationsimpuls elektrische Energie erforderlich ist, die insbesondere im Falle eines Implantats einer Batterie mit begrenzter Kapazität zu entnehmen ist und im Falle einer erschöpften Batterie eine Operation erforderlich ist, besteht grundsätzlich das Bedürfnis, die für einen erfolgreichen Elektrostimulationsimpuls erforderliche Energie so gering wie möglich zu halten, ohne den Erfolg der Elektrostimulation zu gefährden. Der Erfolg der Elektrostimulation, d.h. das Ansprechen des Myocards auf einen Stimulationsimpuls wird üblicherweise mit dem englischen Wort "Capture" bezeichnet.

Um eine Stimulationserfolgskontrolle durchführen zu können, weisen Herzschrittmacher häufig Detektionseinheiten auf, um eine erfolgreiche Elektrostimulation zu detektieren. Eine solche Detektionseinheit wird auch als Capture-Detektor bezeichnet.

Dokument EP 0479215 offenbart einen Elektrostimulator gemäß dem Oberbegriff des Anspruchs 1.

Die vorliegende Erfindung gilt dem Aspekt der Capture-Detektion bei Elektrostimulatoren wie Herzschrittmachern oder dergleichen. Weitere Aspekte der Schrittmachersteuerung, wie beispielsweise das Einstellen einer adäquaten Stimulationsrate oder das Unterbinden (Inhibieren) von Stimulationsimpulsen im Falle von natürlichen Kontraktionen des Herzens stehen nicht im Vordergrund und sind dem Fachmann grundsätzlich bekannt.

Im Zusammenhang mit der Capture-Detektion besteht nach wie vor das Bedürfnis, diese möglichst kurzfristig nach Abgabe eines Stimulationsimpulses durchzuführen, um bei mangelndem Stimulationserfolg kurzfristig einen Back-up-Stimulationsimpuls mit größerer Energie an das Myokard abgeben zu können.

Das vorgenannte Ziel wird erfindungsgemäß durch einen Elektrostimulator der Eingangs genannten Art erreicht, bei dem zwischen dem Elektrodenanschluss für die Aufnahme des ein intrakardiales Elektrokardiogramm repräsentierenden elektrische Signals und der Detektionseinheit ein Hochpassfilter mit einer unteren Grenzfrequenz > 100 Hz angeordnet ist und bei dem die Detektionseinheit ausgebildet ist, das hochpassgefilterte elektrische Signal auszuwerten.

Die Erfindung liegt die Erkenntnis zu Grunde, dass die höherfrequenten Bestandteile eines intrakardialen Elektrokardiogramms vor allem von dem in unmittelbarer Nähe einer Sensing-Elektrode liegenden Myocard bestimmt sind und weniger von ferneren Ereignissen, so dass beispielsweise grundsätzlich bekannte Probleme wie Crosstalk bei der erfindungsgemäßen Anordnung leicht unterdrückt werden können. Außerdem erlaubt die Auswertung des Elektrokardiogramms in unmittelbarer Nähe einer entsprechenden Sensing-Elektrode, einen Stimulationserfolg bereits in einem Zeitraum von wenigen Millisekunden nach Stimulationsimpulsabgabe zu erfassen.

Der Elektrostimulator ist ausgebildet, das ein intrakardiales Elektrokardiogramm repräsentierende elektrische Signal über eine bipolare E-lektrodenkonfiguration aufzunehmen. Hierzu ist an dem Elektrodenanschluss zur Aufnahme des ein intrakardiales Elektrokardiogramm repräsentierenden elektrischen Signals eine wenigstens bipolare Elektrodenleitung angeschlossen, welche wenigstens zwei Elektroden für die Aufnahme an den Elektroden anliegender elektrischer Potentiale aufweist. Die Elektroden weisen vorzugsweise eine geringe Polarisationsneigung auf, d.h. ein geringes Nachpotential nach erfolgter Elektrostimulation. Daher sind insbesondere solche Elektroden bevorzugt, die eine besonders große wirksame Elektrodenoberfläche aufweisen, welche vorzugsweise mit einer fraktalen Beschichtung erzielt wird.

Die Erfindung beruht auf der Erkenntnis, dass bei einer bipolaren Messanordnung unter Verwendung einer Elektrodenleitung mit einer Tip- und einer Ringelektrode für die Aufnahme des intrakardialen Elektrokardiogramms nur kardiale Aktivitäten erfasst werden, die von einem kleinen Gewebebereich um die Elektroden stammen und dass ein derartiges intrakardiales Elektrokardiogramm signifikante Signalkomponenten jenseits von 100 Hz aufweisen. Übliche und bekannte Herzschrittmacher operieren hingegen mit Tiefpassfiltern mit einer oberen Grenzfrequenz in der Größenordnung von 100 Hz, so dass üblicherweise Signalanteile mit Frequenzkomponenten oberhalb von 50 bis 200 Hz unterdrückt werden.

Zum Abbau der durch Polarisation der Stimulationselektroden verursachten Nachpotentiale auf den Stimulationselektroden ist üblicherweise eine sogenannte Autoshort-Periode vorgesehen, innerhalb derer die Elektroden kurzgeschlossen werden, so dass die Nachpotentiale schnell abgebaut werden. Während des Kurzschließens der Stimulationselektroden kann mit diesen Elektroden kein intrakardiales Elektrokardiogramm am Stimulationsort aufgenommen werden. Die üblichen Autoshort-Perioden von 10 bis 20 ms sind zu lang, um bei stark überschwelliger Stimulation eine Reizantwort mittels des intrakardialen Elektrokardiogramms erfassen zu können. Daher wird ein Elektrostimulator bevorzugt, welcher eine Autoshort-Periode von weniger als 5 ms aufweist. Dies lässt sich insbesondere in Verbindung mit fraktal beschichteten Elektroden verwirklichen, die aufgrund der großen Elektrodenoberfläche nur geringe Nachtpotentiale bilden.

Die Detektionseinheit ist in einer bevorzugten Ausführungsvariante dazu ausgebildet, eine erfolgreiche Stimulation, also ein Capture, zu detektieren und daraufhin ein entsprechendes Capture-Signal zu erzeugen. Vorzugsweise geschieht dies dadurch, dass die Detektionseinheit eine erfolgreiche Stimulation anhand eines kurzfristigen Peaks, in dem ein intrakardiales Elektrokardiogramm repräsentierenden elektrischen Signal detektiert und zwar vorzugsweise durch Schwellwertvergleich innerhalb eines definierten Zeitfensters, welches mit Abgabe eines Stimulationsimpulses gestartet wird.

Im Zusammenhang mit einer Detektionseinheit, die ein Capture-Signal erzeugt, ist ein Elektrostimulator bevorzugt, der eine Stimulationssteuereinheit aufweist, die mit der Stimulationseinheit und der Detektionseinheit derart verbunden und ausgebildet ist, dass die Stimulationssteuereinheit aus dem zeitlichen Abstand zwischen Stimulationsimpuls und Capture-Signal ein Stimulationsimpulsstärkensignal ermittelt, welches die Stärke, insbesondere die Amplitude, eines folgenden, durch die Stimulationseinheit erzeugten Stimulationsimpulses bestimmt. Diese bevorzugte Ausführungsvariante beruht auf der nachfolgenden näher erläuterten Erkenntnis, dass die Dauer zwischen Abgabe eines Stimulationsimpulses und Eintreten eines Stimulationserfolges davon abhängt, wie stark überschwellig ein jeweiliger Stimulationsimpuls ist. Während bei einem stark überschwelligen Stimulationsimpuls (Amplitude beispielsweise viermal größer als die Reizschwelle) bereits nach weniger als 5 ms ein Stimulationserfolg eintritt, vergehen bei einem knapp überschwelligen Stimulationsimpuls beispielsweise mehr als 15 ms zwischen Stimulationsimpulsabgabe und Eintritt des Stimulationserfolges. Als Zeitpunkt des Eintritts des Stimulationserfolges wird hierbei der jeweilige charakteristische Peak in dem intrakardial aufgenommenen und hochpassgefilterten Elektrokardiogramm gewertet. Die Erkenntnis, dass gerade ein hochpassgefiltertes intrakardiales Elektrokardiogramm eine genaue Ermittlung des Zeitpunkts des Eintretens eines Stimulationserfolges ermöglicht gepaart mit der Erkenntnis, dass der zeitliche Abstand zwischen Stimulationsimpulsabgabe und Eintreten des Stimulationserfolges kennzeichnend dafür ist, wie stark überschwellig der jeweilige Stimulationsimpuls ist, wird erfindungsgemäß dazu genutzt, die Stimulationsimpulsstärke, d.h. insbesondere die Amplitude eines Stimulationsimpulses jeweils dahingehend einzustellen, dass der Stimulationsimpuls gerade die erforderliche Stärke einschließlich eines eventuell gewünschten Sicherheitszuschlags aufweist ohne unnötig viel Energie zu beanspruchen.

Zu diesem Zweck ist die Stimulationssteuereinheit vorzugsweise ausgebildet, den zeitlichen Abstand zwischen Abgabe eines Stimulationsimpulses und dem darauf folgenden Capture-Signal mit einem Referenzzeitwert zu vergleichen und das Stimulationsimpulsstärkensignal derart einzustellen, dass die Stärke eines jeweiligen Stimulationsimpulses mit geringerem zeitlichen Abstand zwischen Abgabe eines Stimulationsimpulses und dem darauf folgenden Capture-Signal abnimmt, solange der zeitliche Abstand den Referenzzeitwert nicht unterschreitet. Der Referenzzeitwert ist in diesem Zusammenhang vorzugsweise derart eingestellt, dass er einer ausreichend überschwelligen Stimulation entspricht. Das Einstellen des Referenzzeitwertes kann beispielsweise nach der Implantation eines Elektrostimulators durch den betreuenden Arzt erfolgen oder aber auch in einer selbstständigen Lernphase, in der der Elektrostimulator mehrere Stimulationsimpulse unterschwelliger und übeschwelliger Stärke abgibt und die längste, zwischen Stimulationsimpulsabgabe und Eintreten des Stimulationserfolges verstreichende Zeit misst. Diese Zeit entspricht der Latenzzeit zwischen Stimulationsimpulsabgabe und Eintreten des Stimulationserfolges, die dann zu messen ist, wenn die Stimulationsimpulsstärke gerade der Reizschwelle entspricht. Aus Sicherheitsgründen wird der Referenzwert geringfügig kürzer eingestellt, als die so ermittelte Latenzzeit.

Weiterhin ist die Stimulationssteuereinheit vorzugsweise derart ausgebildet, dass sie im Falle des Ausbleibens eines Stimulationserfolges innerhalb eines vorgegebenen Zeitfensters nach Abgabe des Stimulationsimpulses einen Back-up-Stimulationsimpuls auslöst, der eine größere Stärke aufweist, als der zuvor erfolglose Stimulationsimpuls. Auch hier erlaubt die Auswertung des hochpassgefilterten intrakardialen Elektrokardiogramms ein sehr zügiges Auslösen eines Back-up-Stimulationsimpulses im Anschluss an eine vorangegangene erfolglose Stimulation.

Weiterhin ist ein Elektrostimulator bevorzugt, der mit einer Telemetrieeinheit versehen ist, mit welcher sich Werte für den zeitlichen Abstand zwischen einem Stimulationsimpuls und dem daraufhin eintretenden Stimulationserfolg in ein externes Gerät übermitteln lassen. Diese Werte werden vorzugsweise als Wertepaare gemeinsam mit der zugehörigen Stimulationsimpulsstärke übermittelt. Das Aussenden der Zeitabstandswerte und gegebenenfalls der dazugehörigen Stimulationsimpulsstärken kann in Echtzeit erfolgen. Alternativ kann auch eine Speicher vorgesehen sein, in dem mehrere dieser Werte gespeichert werden und entweder zu einem vorgesehenen Zeitpunkt oder auf eine Abfrage hin ausgesandt werden.

Die Erfindung soll nun anhand von Ausführungsbeispielen mit Hilfe der beigefügten Zeichnungen näher erläutert werden. In den Zeichnungen zeigen:
- Figur 1: eine schematische Darstellung eines erfindungsgemäßen Elektrostimulators;
- Figur 2: ein Beispiel für ein intrakardiales Elektrokardiogramm, welches natürliche Herzkontraktionen repräsentiert;
- Figur 3: ein Beispiel eines intrakardialen Elektrokardiogramms, welches Gewebepotentiale nach erfolgreicher Elektrostimulation repräsentiert;
- Figur 4: Aktionspotentiale des Myocards im Anschluss an eine erfolgreiche Elektrostimulation für verschiedene Stimulationsstärken;
- Figur 5: verschiedene Elektrokardiogramme, die die Reizantwort des Myocards bei Stimulation mit verschiedenen Stimulationsstärken repräsentieren;
- Figur 6a: eine schematische Darstellung eines Aktionspotentials; und
- Figur 6b: eine Darstellung der lonenleitfähigkeit und der lonenströme, die zu dem in Figur 6a dargestellten Aktionspotentialverlauf führen.

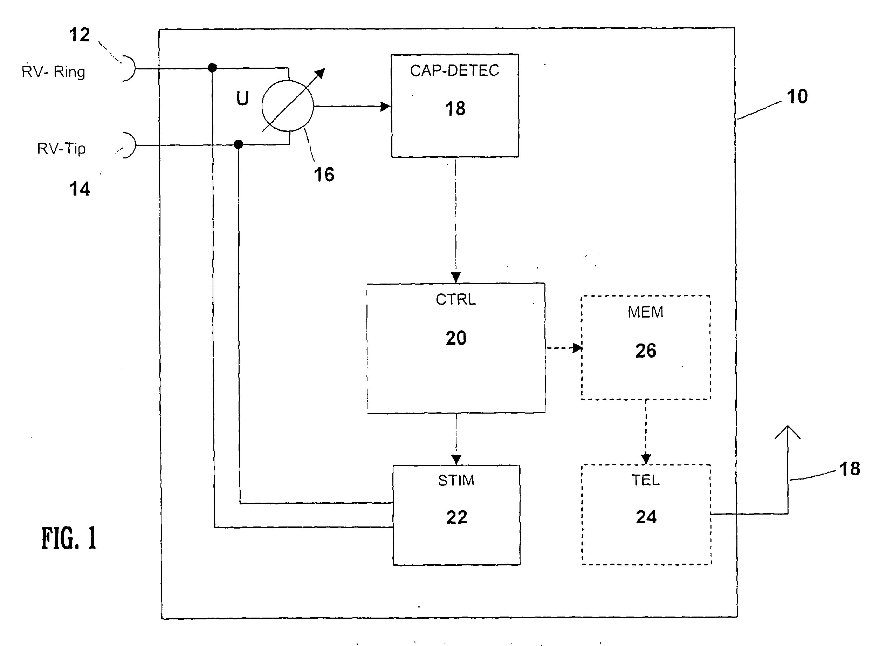

In Figur 1 ist in schematischer Darstellung ein Elektrostimulator 10 dargestellt, der insbesondere ein implantierbarer Herzschrittmacher aber auch ein Cardioverter/Defibrillator oder beides sein kann.

Der Elektrostimulator 10 ist wenigstens mit den beiden in Figur 1 dargestellten Elektrodenanschlüssen 12 und 14 für eine rechtsventrikuläre Ring- und eine rechtsventrikuläre Tipelektrode versehen. Anstelle einer Ring- und einer Tipelektrode können auch zwei Ringelektroden vorgesehen sein. Die Anordnung der Elektroden im rechten Ventrikel entspricht dem häufigsten Anwendungsfall. Eine Anordnung in einer vom Coronar Sinus abzweigenden Coronarvene, also dem linken Ventrikel zugeordnet, oder auch am linken Atrium ist ebenfalls möglich.

Um an den Elektroden anliegende Potentiale zu messen, ist eine Spannungsmesseinheit 16 vorgesehen, welche mit den Elektrodenanschlüssen verbunden ist und ein jeweils ermitteltes Spannungssignal an einen Capture-Dektektor 18 weitergibt. Der Capture-Detektor 18 dient als Detektionseinheit im Sinne der Ansprüche.

Ein in Figur 1 nicht näher dargestellter Hochpassfilter mit einer unteren Grenzfrequenz von ≥ 100 Hz ist entweder der Spannungsmesseinheit 16 vorgeschaltet oder zwischen Spannungs-Messeinheit 16 und Capture-Detektor 18 vorgesehen.

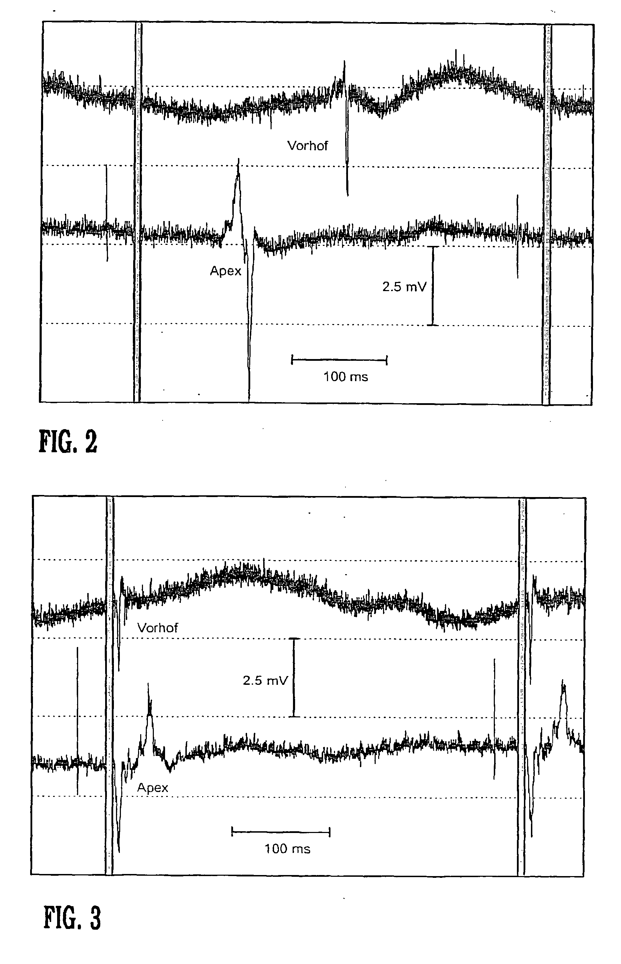

Der Capture-Detektor 18 ist ausgebildet, das hochfrequente intrakardiale Elektrokardiogramm auszuwerten. Beispiele für derartige Elektrokardiogramme im Falle einer natürlichen und einer stimulierten Kontraktion des Herzen sind in Figuren 2 bzw. 3 dargestellt.

Der Capture-Detektor 18 ist ausgebildet, einen für eine erfolgreiche Stimulation charakteristischen Peak in dem hochpassgefilterten Elektrokardiogramm zu detektieren, wie es in Figur 3 dargestellt ist. Der Capture-Detektor ist weiterhin ausgebildet, solche Peaks zu detektieren, die innerhalb eines vorgegebenen Zeitfensters nach Abgabe eines Stimulationsimpulses auftreten. Dazu ist der Capture-Detektor mit einem Timer versehen, der gestartet wird, sobald ein Stimulationsimpuls abgegeben wird.

Der Capture-Detektor 18 ist weiterhin ausgebildet, mit Hilfe des Timers den Zeitabstand zu bestimmen, der zwischen Abgabe eines jeweiligen Stimulationsimpulses und Eintreten des Stimulationserfolges besteht. Der Capture-Detektor leitet aus dem Zeitabstand ein Zeitabstandssignal ab, welches der Capture-Detektor an eine Stimulationssteuereinheit 20 weitergibt.

Außerdem generiert der Capture-Detektor 18 ein Capture-Signal, wenn der Capture-Detektor 18 innerhalb des vorgegebenen Zeitfensters in dem hochpassgefilterten intrakardialen Elektrokardiogramm einen Stimulationserfolg anhand des charakteristischen Peaks erfasst. Im umgekehrten Fall wenn innerhalb des Zeitfensters kein Stimulationserfolg ermittelt wird, generiert der Capture-Detektor 18 ein Non-Capture-Signal. Sowohl das Capture-Signal als auch das Non-Capture-Signal werden vom Capture-Detektor 18 an die Stimulationssteuereinheit 20 weitergegeben.

Die Stimulationssteuereinheit 20 löst im Falle eines Non-Capture-Signals seitens des Capture-Detektors 18 unverzüglich einen Back-up-Stimulationsimpuls aus. Hierzu ist die Stimulationssteuereinheit 20 mit einer Stimulationseinheit 22 verbunden. Der Back-up-Stimulatiosimpuls weist eine größere Stärke, insbesondere eine größere Amplitude auf, als der zuvor erfolglose Stimulationsimpuls.

Im Falle eines Capture-Signals seitens des Capture-Detektors 18 wertet die Stimulationssteuereinheit 20 das Zeitabstandssignal aus und ermittelt, ob und in einer bevorzugten Ausführungsvariante auch um wie viel der zeitliche Abstand zwischen Abgabe eines Stimulationsimpulses und eintreten des Stimulationserfolges (Capture-Signal) kürzer ist als ein zuvor gespeicherter Referenzzeitwert. Solange der zeitliche Abstand, gegeben durch das Zeitabstandsignal, kürzer ist als der Referenzzeitwert, generiert die Stimulationssteuereinheit 20 ein Stimulationsimpulsstärkensignal, welches die Stimulationseinheit 22 dazu veranlasst, den nächstfolgenden Stimulationsimpuls mit geringerer Stärke, d.h. mit geringerer Amplitude abzugeben.

Dies hat aufgrund der zuvor erläuterten physiologischen Zusammenhänge die Folge, dass die sich an den nächst folgenden Stimulationsimpuls anschließende Latenzzeit bis zum Eintreten des Stimulationserfolges verlängert. Wenn die Latenzzeit lang genug ist und dem Referenzzeitwert entspricht, bewirkt die Stimulationssteuereinheit 20 keine weitere Verringerung der Stimulationsimpulsstärke. Auf diese Weise lässt sich eine elegante Regelung der Stimulationsimpulsstärke verwirklichen, die anders als beim Stand der Technik nicht darauf angewiesen ist, regelmäßig unterschwellige, also unwirksame Stimulationsimpulse zu erzeugen.

Weitere in der schematischen Darstellung nicht weiter repräsentierte und dem Fachmann grundsätzlich bekannte Eigenschaften des Elektrostimulators aus Figur 1 sind beispielsweise durch einen Kurzschlussschalter zum Kurzschließen der beiden Elektrodenanschlüsse 12 und 14 gegeben, um das sogenannte Autoshort durchzuführen. Der Kurzschlussschalter wird bei dem erfindungsgemäßen Elektrostimulator für maximal 5 ms nach Abgabe eines Stimulationsimpulses geschlossen, um in direktem Anschluss an diese 5 ms ein intrakardiales Elektroakardiogramm aufnehmen zu können. Dazu muss der Kurzschlussschalter wieder geöffnet sein, d.h. die beiden Elektrodenanschlüsse 12 und 14 sind nicht kurzgeschlossen.

In Figur 1 gestrichelt dargestellt ist eine optionale Telemetrieeinheit 24, die über einen Speicher 26 mit der Stimulationssteuereinheit 20 verbunden ist. Die Telemetrieeinheit 24 dient dazu, in dem Speicher 26 gespeicherte Zeitabstandssignale und dazugehörige Stimulationsimpulsstärken paarweise an ein externes Gerät telemetrisch zu übermitteln. Die Telemetrieeinheit 24 ist mit einer in Figur 1 angedeuteten Antenne 28 verbunden.

Die Analyse der Zeitabstandswerte und der dazugehörigen Stimulationsamplituden kann dann auch in einem externen Gerät erfolgen, um beispielsweise einen geeigneten Referenzzeitwert zu ermitteln. Dies erlaubt, die Elektrostimulator-interne Stimulationssteuereinheit einfacher zu gestalten, als zuvor beschrieben.

Figur 2 zeigt eine intrakardiales Elektrokardiogramm eines intrinsischen Ereignisses wie es mit einer natürlichen Herzkontraktion einhergeht. Das intrakardiale Elektrokardiogramm ist hochpassgefiltert mit einem Hochpassfilter erster Ordnung und einer Grenzfrequenz von 500 Hz. Die zwei senkrechten Balken repräsentieren Perioden, in denen das entsprechende Herz nicht wirksam mit einem Schrittmacher stimuliert wurde und die daher von der Darstellung ausgenommen wurden.

Die Aufnahme des intrakardialen Elektrokardiogramms erfolgt mit einer bipolaren Messanordnung, bei der die Ring- und die Tipelektrode einer einzelnen Elektrodenleitung für die Aufnahme der Gewebepotentiale verwendet wurden. Die aufgenommen Gewebepotentiale wurden hochpassgefiltert. Dies resultierte in den in Figur 2 dargestellten scharfen Signalspitzen. Diese Signalspitzen gehen mit einer Anstiegsphase eines lokalen Aktionspotentials einher.

Die bipolare Messanordnung stellt sicher, dass nur elektrische Aktivitäten aus der nächsten Umgebung der Elektroden gemessen werden. Aktivitäten anderer Herzkammern oder der Skelettmuskeln sind stark unterdrückt. Dieses reduziert sowohl eine mögliche Crosstalk-Wahrnehmung (Wahrnehmung von Aktivitäten anderer Herzkammern als der, in der gemessen wird) als auch das Erfassen von muskulären Aktivitäten. Dieser Effekt ist durch die Hochpassfilterung des Signals weiter verstärkt. In einer bipolaren Messanordnung verschiebt sich die Kurve der Frequenzantwort umso mehr in Richtung niedrigerer Frequenzen, je größer der Messabstand wird. Mit zunehmender Entfernung vom Messort werden höhere Frequenzkomponenten stärker verringert als niederfrequente Signalkomponenten. Daher wird durch die Hochpassfilterung des Messsignal Crosstalk und die Wahrnehmung von Skelettmuskelaktivitäten noch weiter reduziert als durch die bipolare Messanordnung bereits gegeben.

Unmittelbar nach der Abgabe von Stimulationsimpulsen schließt ein Herzschrittmacher die Stimulationselektroden üblicherweise kurz (während einer sogenannten Autoshort-Periode) um Ladungen abfließen zu lassen, die sich während der Stimulation auf der Elektrodenoberfläche angesammelt haben. Die Dauer dieser Autoshort-Periode beträgt üblicherweise 10 bis 20 ms, so dass die ansteigende Phase von Aktionspotentialen, die auf einen Stimulationsimpuls zurückgehen nicht wahrgenommen werden können. Daher ist der Herzstimulator in einer bevorzugten Ausführungsvariante dazu ausgebildet, eine Autoshort-Periode von nicht mehr als 5 ms zu schalten. Um dennoch Nachpotentiale auf den Elektroden möglichst gering zu halten, sind diese mit einer möglichst großen aktiven Oberfläche versehen, die durch eine fraktale Beschichtung erreicht wird.

Figur 3 zeigt ein hochpassgefiltertes intrakardiales Elektrokardiogramm in Reaktion auf einen Stimulationsimpuls. Ein den Stimulationserfolg kennzeichnender scharfer negativer Peak folgt innerhalb von 15 ms auf den Stimulationsimpuls.

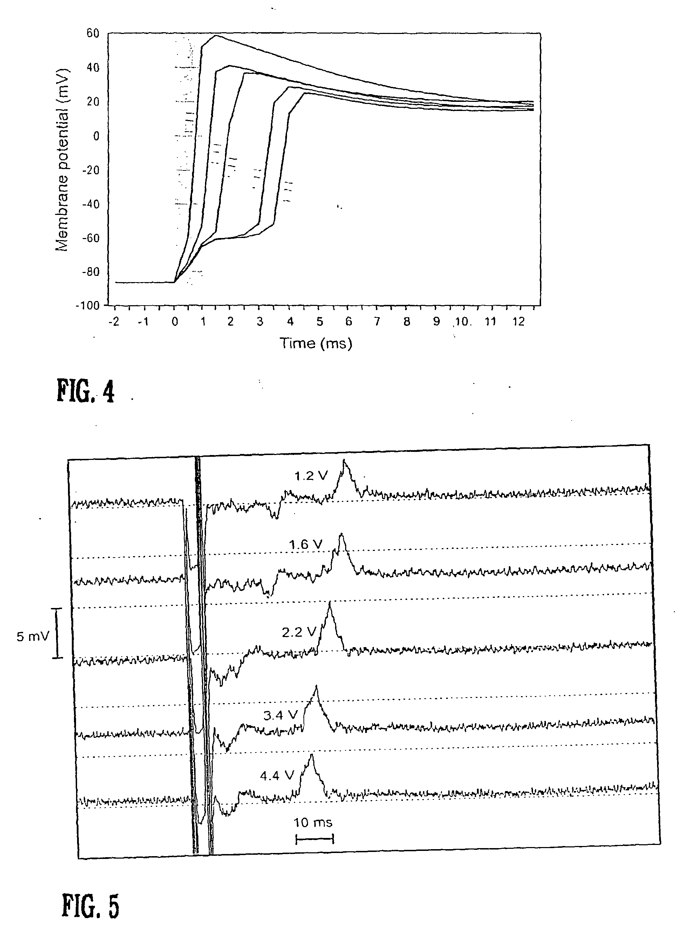

Figur 4 zeigt den typischen Verlauf von Aktionspotentialen der Zellmembranen des Myocards im Anschluss an eine Elektrostimulation. Gut zu erkennen sind die ansteigenden Phasen des Verlaufs der Aktionspotentiale. Die Aktionspotentiale mit der am weitesten links gelegenen Anstiegsphase gehen auf Stimulationsimpulse größerer Stärke (größerer Spannung, Energie) zurück, die weiter rechts gelegenen Aktionspotentialverläufe auf eine Stimulation mit schwächeren Stimulationsimpulsen. Die Verläufe der Aktionspotentiale ähneln sich. Jedoch ist bei Stimulationsimpulsen nahe der Reizschwelle des Myocards (schwach überschwellig) zu erkennen, dass das Membranpotential zunächst für wenige Millisekunden auf einem geringfügig erhöhten Plateau verharrt, bevor die eigentliche Anstiegsphase des Aktionspotentialverlaufs eintritt.

Diese Anstiegsphase des Aktionspotentialverlaufs spiegelt sich in einem Peak in einem entsprechenden hochpassgefilterten intrakardialen Elektrokardiogramms wieder. Derartige Elektrokardiogramme für verschiedene Stimutationsimpulsstärken sind in Figur 5 wiedergegeben. Zu erkennen ist, dass der Peak im intrakardialen Elektrokardiogramm umso später auftritt, je geringer der Stimulationsimpuls ist. Diese Zeitverschiebung zwischen dem Auftreten eines Peaks im intrakardialen Elektrokardiogramm für einen Stimulationsimpuls der gerade überschwellig ist, und einem, der viermal so groß ist, wie die Reizschwelle des Myocards, beträgt etwa 10 bis 15 ms.

Die Position eines Peaks im intrakardialen Elektrokardiogramm ist daher ein Indikator dafür, um wie viel die Amplitude eines Stimulationsimpulses oberhalb der Reizschwelle liegt. In einer bevorzugten Ausführungsvariante ist der beanspruchte Elektrostimulator daher ausgebildet, die Stärke eines Stimulationsimpulses in Abhängigkeit der Dauer einzustellen, die zwischen Abgabe eines Stimulationsimpulses und Auftreten des Peaks im hochpassgefilterten intrakardialen Elektrokardiogramm verstreicht. Ist diese Zeitdauer sehr kurz, beispielsweise kleiner als 5 bis 10 ms, kann die Stärke des Stimulationsimpulses verringert werden, ohne den Stimulationserfolg für den nächsten Stimulationsimpuls zu gefährden. Dies steht im Gegensatz zu anderen bekannten Herzschrittmachern mit einem sogenannten Auto-Capture-Algorithmus, bei dem die Stimulationsimpulsamplitude regelmäßig so lange verringert wird, bis kein Stimulationsimpuls mehr feststellbar ist. Erst dann wird die Stimulationsimpulsamplitude wieder geringfügig angehoben. Somit nehmen bekannte Auto-Capture-Algorithmen regelmäßig einen mangelnden Stimulationserfolg in Kauf.

Der bevorzugte Elektrostimulator stellt daher vorzugsweise in einer anfänglichen Lernphase automatisch einen Referenzzeitwert für den zeitlichen Abstand zwischen Stimulationsimpulsabgabe und Auftreten der Reizantwort ein, unterhalb dessen eine erfolgreiche Stimulation regelmäßig sichergestellt ist. Während des nachfolgenden Betriebes stellt die Stimulationssteuereinheit des Herzschrittmachers die Stimulationsstärke regelmäßig so ein, dass der jeweils gemessene Zeitwert geringfügig unterhalb des Referenzzeitwertes liegt oder diesem entspricht.

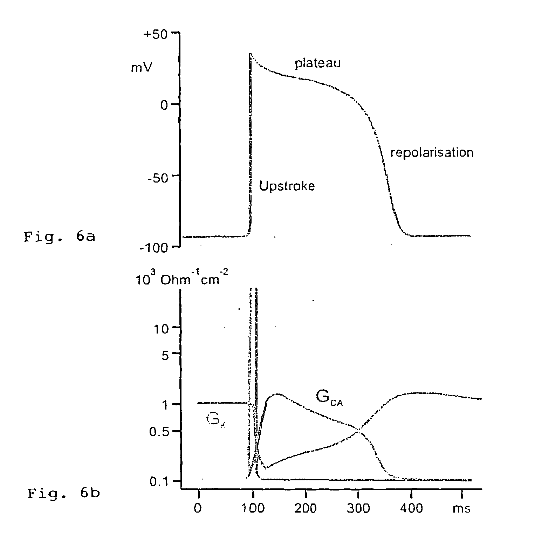

Die Figuren 6a und 6b dienen der weiteren Erläuterung des Verhaltens des Myocards im Anschluss an eine Elektrostimulation.

## Patentansprüche

1. Elektrostimulator mit Elektrodenanschlüssen (12, 14), die wenigstens zeitweise mit einer Stimulationseinheit (22) und einer Detektionseinheit (18) des Elektrostimulators zu verbinden sind, wobei die Stimulationseinheit (22) ausgebildet ist, Elektrostimulationsimpulse zur Stimulation von Körpergewebe zu generieren und an wenigstens einen der Elektrodenanschlüsse (12, 14) abzugeben und wobei die Detektionseinheit (18) dazu ausgebildet ist, eine erfolgreiche Stimulation von Körpergewebe anhand eines an wenigstens einem Elektrodenanschluss anliegendem elektrischen Signal zu detektieren, wobei der Elektrostimulator ausgebildet ist, über eine biopolare Elektrodenkonfiguration ein intrakardiales Elektrokardiogramm repräsentierendes elektrisches Signal aufzunehmen,
**dadurch gekennzeichnet, dass** zwischen dem Elektrodenleitungsanschluss und der Detektionseinheit ein Hochpassfilter mit einer unteren Grenzfrequenz größer 100 Hz angeordnet ist und die Detektionseinheit ausgebildet ist, das hochpassgefilterte elektrische Signal auszuwerten.

2. Elektrostimulator nach Anspruch 1, **dadurch gekennzeichnet, dass** an dem Elektrodenanschluss zur Aufnahme des ein intrakardiales Elektrokardiogramm repräsentierenden elektrischen Signals eine wenigstens bipolare Elektrodenleitung angeschlossen ist, welche wenigstens zwei Elektroden für die Aufnahme an den E-lektrodenanliegender elektrischer Potentiale aufweist, wobei die Elektroden eine geringe Polarisation aufweisen.

3. Elektrostimulator nach Anspruch 2, **dadurch gekennzeichnet, dass** die Elektroden eine fraktale Beschichtung aufweisen.

4. Elektrostimulator nach einem der Ansprüche 1 bis 3, **dadurch gekennzeichnet, dass** der Elektrostimulator eine Autoshorteinrichtung zum Kurzschließen von Stimulationselektroden aufweist, die ausgebildet ist, die Elektroden nicht länger als 5 Millisekunden kurzzuschließen.

5. Elektrostimulator nach einem der Ansprüche 1 bis 4, **dadurch gekennzeichnet, dass** die Detektionseinheit ausgebildet ist, eine erfolgreiche Stimulation (capture) zu detektieren und ein Capture-Signal zu erzeugen.

6. Elektrostimulator nach Anspruch 5, **dadurch gekennzeichnet, dass** die Detektionseinheit ausgebildet ist, eine erfolgreiche Stimulation anhand eines kurzfristigen Peaks in dem ein intrakardiales Elektrokardiogramm repräsentierenden elektrischen Signal zu detektieren.

7. Elektrostimulator nach Anspruch 6, **dadurch gekennzeichnet, dass** die Detektionseinheit ausgebildet ist, einen kurzfristigen Peak in dem ein intrakardiales Elektrokardiogramm repräsentierenden elektrischen Signal innerhalb eines definierten Zeitfensters zu detektieren, welches mit Abgabe eines Stimulationsimpulses gestartet wird.

8. Elektrostimulator nach Anspruch 7 und einem der übrigen Ansprüche, **gekennzeichnet durch** eine Stimulationssteuereinheit, die mit der Stimulationseinheit und der Detektionseinheit derart verbunden und ausgebildet ist, dass die Stimulationssteuereinheit aus dem zeitlichen Abstand zwischen Stimulationsimpuls und Capture Signal ein Stimulationsimpulsstärkensignal ermittelt, welches die Stärke eines folgenden, **durch** die Stimulationseinheit erzeugten Stimulationsimpulses bestimmt.

9. Elektrostimulator nach Anspruch 8, **dadurch gekennzeichnet, dass** die Stimulationssteuereinheit ausgebildet ist, den zeitlichen Abstand zwischen Abgabe eines Stimulationsimpulses und dem darauf folgenden Capture-Signal mit einem Referenzzeitwert zu vergleichen, und das Stimulationsimpulsstärkensignal derart einzustellen, dass die Stärke eines Stimulationsimpulses mit geringerem zeitlichen Abstand zwischen Abgabe eines Stimulationsimpulses und dem darauf folgenden Capture-Signal abnimmt, solange der zeitliche Abstand den Referenzzeitwert unterschreitet.

10. Elektrostimulator nach Anspruch 9, **dadurch gekennzeichnet, dass** der Referenzzeitwert derart eingestellt ist, dass er einer ausreichend überschwelligen Stimulation entspricht.

11. Elektrostimulator nach Anspruch 8, **dadurch gekennzeichnet, dass** die Stimulationssteuereinheit ausgebildet ist, im Falle des Ausbleibens eines Capture-Signals innerhalb eines vorgegebenen Zeitfensters nach Abgabe eines Stimulationsimpulses einen Backup-Stimulationsimpuls größerer Stärke auszulösen.

12. Elektrostimulator nach Anspruch 5 und einem der übrigen Ansprüche, **gekennzeichnet durch** eine Telemetrieeinheit, die mit der Detektionseinheit verbunden und ausgebildet ist, ein dem zeitlichen Abstand zwischen Stimulationsimpuls und Capture Signal entsprechendes Zeitabstandssignal an ein externes Gerät zu senden.

13. Elektrostimulator nach Anspruch 12, **gekennzeichnet durch** einen Speicher für einen oder mehrere Werte des Zeitanstandssignals, der mit der Detektionseinheit und der Telemetrieeinheit verbunden ist.

## Claims

1. An electrostimulator having electrode terminals (12, 14), which are to be connected at least sometimes to a stimulation unit (22) and a detection unit (18) of the electrostimulator, the stimulation unit (22) being implemented to generate electrostimulation pulses for stimulating body tissue and deliver them to at least one of the electrode terminals (12, 14), and the detection unit (18) being implemented to detect a successful stimulation of body tissue on the basis of at least one electrical signal applied to an electrode terminal, the electrostimulator being implemented to record an electrical signal representing an intracardial electrocardiogram via a bipolar electrode configuration, **characterized in that** a high-pass filter having a lower limiting frequency greater than 100 Hz is situated between the electrode line terminal and the detection unit, and the detection unit is implemented to analyze the high-pass-filtered electrical signal.

2. The electrostimulator according to Claim 1, **characterized in that** an at least bipolar electrode line, which has at least two electrodes for recording electrical potentials applied to the electrodes, the electrodes having a slight polarization, is connected to the electrode terminal to record the electrical signal representing an intracardial electrocardiogram.

3. The electrostimulator according to Claim 2, **characterized in that** the electrodes have a fractal coating.

4. The electrostimulator according to one of Claims 1 through 3, **characterized in that** the electrostimulator has an autoshort unit for short-circuiting stimulation electrodes, which is implemented to short-circuit the electrodes not longer than 5 ms.

5. The electrostimulator according to one of Claims 1 to 4, **characterized in that** the detection unit is implemented to detect a successful stimulation (capture) and generate a capture signal.

6. The electrostimulator according to Claim 5, **characterized in that** the detection unit is implemented to detect a successful stimulation on the basis of a brief peak in the electrical signal representing an intracardial electrocardiogram.

7. The electrostimulator according to Claim 6, **characterized in that** the detection unit is implemented to detect a brief peak in the electrical signal representing an intracardial electrocardiogram within a defined time window, which is started upon delivery of a stimulation pulse.

8. The electrostimulator according to Claim 7 and one of the remaining claims, **characterized by** a stimulation control unit, which is connected to the stimulation unit and the detection unit and implemented in such a way that the stimulation control unit ascertains a stimulation pulse strength signal from the chronological interval between stimulation pulse and capture signal, which determines the strength of a following stimulation pulse generated by the stimulation unit.

9. The electrostimulator according to Claim 8, **characterized in that** the stimulation control unit is implemented to compare the chronological interval between delivery of the stimulation pulse and the following capture signal to a reference time value, and to set the stimulation pulse strength signal in such a way that the strength of a stimulation pulse is reduced upon lower chronological interval between delivery of a stimulation pulse and the following capture signal as long as the chronological interval falls below the reference time value.

10. The electrostimulator according to Claim 9, **characterized in that** the reference time value is set in such a way that it corresponds to a stimulation sufficiently above the threshold.

11. The electrostimulator according to Claim 8, **characterized in that** the stimulation control unit is implemented to trigger a backup stimulation pulse of greater strength if a capture signal does not arrive within a predefined time window after delivery of a stimulation pulse.

12. The electrostimulator according to Claim 5 and one of the remaining claims, **characterized by** a telemetry unit, which is connected to the detection unit and implemented to transmit a time interval signal corresponding to the chronological interval between stimulation pulse and capture signal to an external device.

13. The electrostimulator according to Claim 12, **characterized by** a memory for one or more values of the time interval signal, which is connected to the detection unit and the telemetry unit.

## Revendications

1. Stimulateur électrique avec des branchements d'électrodes (12, 14), qui doivent être reliés au moins de façon temporaire à une unité de stimulation (22) et une unité de détection (18) du stimulateur électrique, l'unité de stimulation (22) étant conçue pour générer des impulsions de stimulation électrique pour la stimulation de tissu du corps et de les envoyer à au moins l'un des branchements d'électrodes (12, 14) et l'unité de détection (18) étant conçue pour détecter une stimulation réussie de tissu du corps à l'aide d'un signal électrique appliqué sur au moins un branchement d'électrodes, le stimulateur électrique étant conçu pour réceptionner par une configuration d'électrodes bipolaire un signal électrique représentant un électrocardiogramme intracardiaque, **caractérisé en ce que** entre le branchement de ligne d'électrodes et l'unité de détection est disposé un filtre passe-haut avec une fréquence limite inférieure supérieure à 100 Hz et l'unité de détection est conçue pour analyser le signal électrique filtré avec un filtre passe-haut.

2. Stimulateur électrique selon la revendication 1, **caractérisé en qu'**au branchement d'électrodes, pour l'enregistrement du signal électrique représentant un électrocardiogramme intracardiaque, est raccordée une ligne d'électrodes au moins bipolaire qui présente au moins deux électrodes pour l'enregistrement de potentiels électriques appliqués sur les électrodes, les électrodes présentant une faible polarisation.

3. Stimulateur électrique selon la revendication 2, **caractérisé en ce que** les électrodes présentent un revêtement fractal.

4. Stimulateur électrique selon l'une quelconque des revendications 1 à 3, **caractérisé en ce que** le stimulateur électrique présente un dispositif autoshort pour le court-circuitage d'électrodes de simulation qui est conçu pour court-circuiter les électrodes pendant une durée inférieure à 5 millisecondes.

5. Stimulateur électrique selon l'une quelconque des revendications 1 à 4, **caractérisé en ce que** l'unité de détection est conçue pour détecter une stimulation réussie (capture) et un signal de capture.

6. Stimulateur électrique selon la revendication 5, **caractérisé en ce que** l'unité de détection est conçue pour détecter une stimulation électrique à l'aide d'un pic de courte durée dans le signal électrique représentant un électrocardiogramme intracardiaque.

7. Stimulateur électrique selon la revendication 6, **caractérisé en ce que** l'unité de détection est conçue pour détecter un pic de courte durée dans le signal électrique représentant un électrocardiogramme intracardiaque à l'intérieur d'une fenêtre de temps définie qui est démarrée avec l'envoi d'une impulsion de stimulation.

8. Stimulateur électrique selon la revendication 7 et l'une quelconque des autres revendications, **caractérisé par** une unité de commande de stimulation, qui est reliée à l'unité de stimulation et à l'unité de détection et conçue de telle sorte que l'unité de commande de stimulation détermine à partir de l'intervalle de temps entre l'impulsion de stimulation et le signal de capture un signal d'intensité d'impulsion de stimulation qui détermine l'intensité d'une impulsion de stimulation suivante et générée par l'unité de stimulation.

9. Stimulateur électrique selon la revendication 8, **caractérisé en ce que** l'unité de commande de stimulation est conçue pour comparer l'intervalle de temps entre l'envoi d'une impulsion de stimulation et le signal de capture consécutif avec une valeur de temps de référence, et régler le signal d'intensité d'impulsion de stimulation de telle sorte que l'intensité d'une impulsion de stimulation est obtenue avec un intervalle de temps plus faible entre l'envoi d'une impulsion de stimulation et le signal de capture consécutif aussi longtemps que l'intervalle de temps est inférieur à la valeur du temps de référence.

10. Stimulateur électrique selon la revendication 9, **caractérisé en ce que** la valeur de temps de référence est réglée de telle sorte qu'elle correspond à une stimulation suffisamment supérieure au seuil.

11. Stimulateur électrique selon la revendication 8, **caractérisé en ce que** l'unité de commande de stimulation est conçue pour déclencher une impulsion de stimulation de sauvegarde d'intensité plus grande en cas d'absence d'un signal de capture à l'intérieur d'une fenêtre de temps prédéfinie après l'envoi d'une impulsion de stimulation.

12. Stimulateur électrique selon la revendication 5 et l'une quelconque des autres revendications, **caractérisé par** une unité de télémétrie qui est reliée à l'unité de détection et conçue pour envoyer un signal d'intervalle de temps correspondant à l'intervalle de temps entre l'impulsion de stimulation et le signal de capture à un appareil externe.

13. Stimulateur électrique selon la revendication 12, **caractérisé par** une mémoire pour une ou plusieurs valeurs du signal d'intervalle de temps, qui est reliée à l'unité de détection et à l'unité de télémétrie.
